# EUROPEAN PATENT APPLICATION

(11) **EP 2 568 293 A1**
(43) Date of publication of application: **13.03.2013**
(21) Application number: 12181017.0
(22) Date of filing: 20.08.2012
(51) Int. Cl.: G01N 33/68, H01J 49/16

(54) **Matrix additive for mass spectrometry**

(30) Priority: 09.09.2011 JP 2011196776; 29.03.2012 JP 2012076289
(71) Applicant: Shimadzu Corporation, Kyoto-shi, Kyoto 604 (JP); Hiroshima University, Hiroshima 739-8511 (JP)
(72) Inventor: Fukuyama, Yuko, Kyoto-shi, Kyoto 604-8511 (JP); Izumi, Shunsuke, Higashi-Hiroshima-shi, Hiroshima 739-8526 (JP)
(74) Representative: Kilian Kilian & Partner

(57) **Abstract**

The application is directed to MALDI matrix additives based on 5-alkoxy-2- or -3-hydroxybenzoic acid represented by the following formula (I): 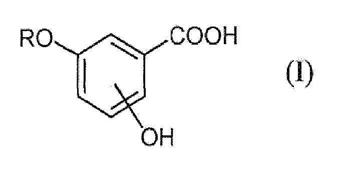 where R is an alkyl group having 6 to 10 carbon atoms and the substituted carboxyl group and hydroxyl group are ortho or meta to each other. A preferred embodiment comprises 5-octyloxysalicylic acid. By including the additive in a matrix for mass spectrometry selected from the group consisting of α-cyano-4-hydroxycinnamic acid, 2,5-dihydroxybenzoic acid, sinapic acid, and 1,5-diaminonaphthalene, the ionization efficiency of hydrophobic peptides is increased, leading to improved MALDI-MS analysis of hydrophobic proteins.

SEQUENCE LISTING

<110> SHIMADZU CORPORATION
HIROSHIMA UNIVERSITY

<120> Matrix Additive for Mass Spectrometry

<130> G2412

<150> JP 2011-196776
<151> 2011-09-09

<150> JP 2012-076289
<151> 2012-03-29

<160> 1

<170> PatentIn version 3.5

<210> 1
<211> 13
<212> PRT
<213> human

<400> 1

Gly Pro His Arg Ser Thr Pro Glu Ser Arg Ala Ala Val
1               5                   10

SEQUENCE LISTING

<110>  SHIMADZU CORPORATION
       HIROSHIMA UNIVERSITY

<120>  Matrix Additive for Mass Spectrometry

<130>  G2412

<150>  JP 2011-196776
<151>  2011-09-09

<150>  JP 2012-076289
<151>  2012-03-29

<160>  1

<170>  PatentIn version 3.5

<210>  1
<211>  13
<212>  PRT
<213>  human

<400>  1

Gly Pro His Arg Ser Thr Pro Glu Ser Arg Ala Ala Val
1               5                   10

## Description

### BACKGROUND OF THE INVENTION

### Field of the Invention

The present invention relates to MALDI-MS (Matrix-Assisted Laser Desorption/Ionization Mass Spectrometry) application. More specifically, the present invention relates to a matrix additive for mass spectrometry.

### Description of Related Art

JP-A-2005-326391 discloses a method for more efficiently ionizing a hydrophobic peptide, especially a peptide modified with a 2-nitrobenzenesulfenyl group (NBS-modified peptide), by using, as a matrix, α-cyano-3-hydroxycinnamic acid (3-CHCA), 3-hydroxy-4-nitrobenzoic acid (3H4NBA), or a mixture of them than by using a common matrix such as α-cyano-4-hydroxycinnamic acid (4-CHCA) or 2,5-dihydroxybenzoic acid (DHB).

### SUMMARY OF THE INVENTION

It is an object of the present invention to provide, as a matrix additive, a novel compound that makes it possible to achieve an analytical method capable of easily and efficiently improving ionization efficiency in mass spectrometry without sample modification (more specifically, without labeling or the like).

The present inventors have intensively studied, and as a result, have found that a compound resembling 2,5-dihydroxybenzoic acid and 3,5-dihydroxybenzoic acid and having an alkyl group with a certain degree of length does not function as a matrix, but the object of the present invention can be achieved by using such a compound as a matrix additive. This finding has led to the completion of the present invention.

The present invention includes the following.
(1)
   A matrix additive for mass spectrometry, which is 5-alkoxy-2- or -3-hydroxybenzoic acid represented by the following formula (I): 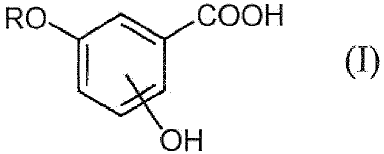 where R is an alkyl group having 6 to 10 carbon atoms and the substituted carboxyl group and hydroxyl group are ortho or meta to each other.
(2)
   The additive according to (1), which is added to a matrix for mass spectrometry selected from the group consisting of α-cyano-4-hydroxycinnamic acid, 2,5-dihydroxybenzoic acid, sinapic acid, and 1,5-diaminonaphthalene. (3)
   The additive according to (1) or (2), which is used for mass spectrometry of a hydrophobic substance.
(4)
   The additive according to (3), wherein the hydrophobic substance is a hydrophobic peptide.
   It is to be noted that in the present invention, the term "peptide" includes proteins.
(5)
   The additive according to (3) or (4), wherein the hydrophobic substance has an HPLC Index of 100 to 10,000.
(6)
   The additive according to any one of (1) to (5), which is used in a molar ratio of 0.01 to 50 moles per mole of a matrix for mass spectrometry.
(7)
   A method for forming a crystal for mass spectrometry, comprising the steps of: preparing, on a target plate for mass spectrometry, a liquid droplet of a mixture containing, in a solvent, at least a hydrophobic substance, a matrix, and the additive according to any one of (1) to (6); and removing the solvent from the liquid droplet.
(8)
   A crystal for mass spectrometry formed on a target plate for mass spectrometry, the crystal for mass spectrometry comprising at least a hydrophobic substance, a matrix, and the additive according to any one of (1) to (6) and having a substantially circular shape with an average diameter of 1 to 3 mm on a surface in contact with the target plate for mass spectrometry, wherein 50 mol% or more of the hydrophobic substance is localized in an outer peripheral region of the substantially circular shape, the average diameter is an average outer diameter of the outer peripheral region, and the outer peripheral region has an average inner diameter that is 80% or more of the average outer diameter.
   The crystal for mass spectrometry according to the above (8) can be formed by the method according to (7).
(9)
   A mass spectrometry method comprising the steps of: preparing, on a target plate for mass spectrometry, a liquid droplet of a mixture containing, in a solvent, at least a hydrophobic substance, a matrix, and the additive according to any one of (1) to (6); removing the solvent from the liquid droplet to form a crystal for mass spectrometry; and irradiating the crystal for mass spectrometry with laser to detect the hydrophobic substance.
(10)
   The mass spectrometry method according to (9), wherein an outer peripheral region of the crystal for mass spectrometry, whose average inner diameter is 80% or more of its average outer diameter, is irradiated with the laser.
   In the method according to the above (10), the outer peripheral region, in which the hydrophobic material is localized, is irradiated with the laser.
(11)
   The mass spectrometry method according to (9) or (10), wherein the mixture further contains a hydrophilic substance.
   A matrix composition for mass spectrometry comprising: a matrix additive for mass spectrometry which is 5-alkoxy-2-or -3-hydroxybenzoic acid represented by the following formula (I): 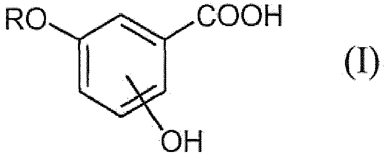 where R is an alkyl group having 6 to 10 carbon atoms and the substituted carboxyl group and hydroxyl group are ortho or meta to each other; and a matrix for mass spectrometry selected from the group consisting of α-cyano-4-hydroxycinnamic acid, 2,5-dihydroxybenzoic acid, sinapic acid, and 1,5-diaminonaphthalene.
(12)
   5-Alkoxy-2- or -3-hydroxybenzoic acid represented by the following formula (I): 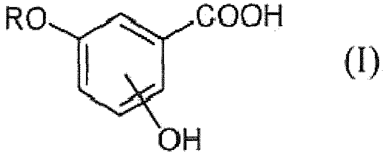
where R is an alkyl group having 6 to 10 carbon atoms and the substituted carboxyl group and hydroxyl group are ortho or meta to each other.

According to the present invention, it is possible to provide a matrix additive capable of improving ionization efficiency of a hydrophobic substance.

The present invention makes it possible to achieve improvement in sensitivity for detection of a hydrophobic substance in mass spectrometry.

### BRIEF DESCRIPTION OF THE DRAWINGS

Fig. 1 is a ¹H NMR spectrum of an example of a compound according to the present invention;
Fig. 2 shows the structure of the example of the compound according to the present invention and the result of proton assignment based on Fig. 1;
Fig. 3 is a mass spectrum of the example of the compound according to the present invention;
Fig. 4 shows the structure of the example of the compound according to the present invention and the result of mass spectrometry based on Fig. 3;
Fig. 5 is a ¹H NMR spectrum of another example of the compound according to the present invention;
Fig. 6 shows the structure of the another example of the compound according to the present invention and the result of proton assignment based on Fig. 5;
Fig. 7 (a) is a mass spectrum of a mixture of a hydrophilic peptide β-amyloid 1-11 and a hydrophobic peptide Humanin, which was obtained in Example 6 when a compound ADHB according to the present invention was used as a matrix additive for mass spectrometry and Fig. 7(b) is a mass spectrum of the mixture, which was obtained in Example 6 when the compound ADHB was not used;
Fig. 8 shows photographs of a crystal formed in a well on a MALDI plate in case of Fig. 7, MS images of the hydrophobic peptide Humanin, and MS images of the hydrophilic peptide β-amyloid 1-11;
Fig. 9(a) is a mass spectrum of a tryptic digest of Phosphorylase b, which was obtained in Example 7 when a compound ADHB according to the present invention was used as a matrix additive for mass spectrometry and in which MS images of peak substances at m/z 3715.2 and m/z 4899.3 are inserted and Fig. 9(b) is a mass spectrum of the tryptic digest of Phosphorylase b, which was obtained in Example 7 when the compound ADHB was not used; and
Fig. 10 shows MS images of various analytes different in HPLC index obtained in Example 8 in a case (a) that a compound ADHB according to the present invention was used as a matrix additive for mass spectrometry and in a case (b) that a compound ADHB was not used, and a sensitivity improvement degree when each of the analytes was analyzed in a case (a) as compared to in a case (b).

### DETAILED DESCRIPTION OF PREFERRED EMBODIMENTS

### [1. 5-Alkoxy-2- or -3-Hydroxybenzoic Acid]

The present invention provides 5-alkoxy-2- or -3-hydroxybenzoic acid represented by the following formula (I): 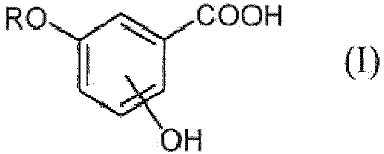 where R is an alkyl group having 6 to 10 carbon atoms. The alkyl group may be linear or branched. The substituted carboxyl group and hydroxyl group are ortho or meta to each other.

The compound represented by the above formula (I) may be synthesized by, for example, introducing an alkyl group into 2,5-dihydroxybenzoic acid or 3,5-dihydroxybenzoic acid using an alkylating agent represented by R-X (where R is an alkyl group having 6 to 10 carbon atoms and X is a halogen (F, Cl, Br or I) or another leaving group).

In this case, the alkylating agent R-X may be used in an amount of 1.0 to 2.0 equivalents per equivalent of 2,5-dihydroxybenzoic acid or 3,5-dihydroxybenzoic acid. As a solvent, acetone, tetrahydrofuran, dimethylformamide, or the like may be used. Reaction conditions may be 2 hours or longer at 50 to 170°C (e.g., acetone: 56°C, tetrahydrofuran: 66°C, dimethylformamide: 153°C).

### [2. Matrix Additive for Mass Spectrometry]

The compound represented by the above formula (I) is useful as a matrix additive for matrix-assisted laser desorption/ionization (MALDI) mass spectrometry. The compound according to the present invention does not have the ability to ionize an analyte alone and therefore does not function as a matrix. However, the use of the compound according to the present invention in combination with a matrix makes it possible to enhance the ability of the matrix to ionize an analyte, thereby improving the limit of detection.

### [3. Matrix]

A matrix to be used in combination with the additive according to the present invention is not particularly limited. The matrix may be appropriately selected from common matrixes by those skilled in the art. For example, the matrix may be selected from the group consisting of
α -cyano-4-hydroxycinnamic acid, 2,5-dihydroxybenzoic acid, sinapic acid, and 1,5-diaminonaphthalene.

Particularly, 5-alkoxy-2-hydroxybenzoic acid represented by the following formula (II), where the substituted carboxyl group and hydroxyl group are ortho to each other, may be preferably used in combination with a matrix for mass spectrometry selected from the group consisting of α-cyano-4-hydroxycinnamic acid, sinapic acid, and 1,5-diaminonaphthalene.

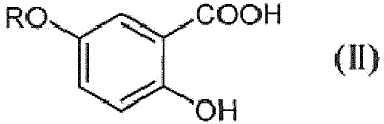

Further, 5-alkoxy-3-hydroxybenzoic acid represented by the following formula (III), where the substituted carboxyl group and hydroxyl group are meta to each other, may be preferably used in combination with a matrix for mass spectrometry selected from the group consisting of 2,5-dihydroxybenzoic acid and 1,5-diaminonaphthalene.

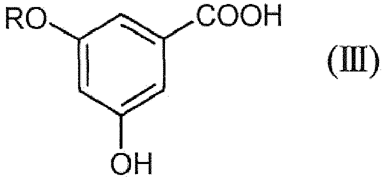

### [4. Object to be Analyzed by Mass Spectrometry]

An object to be analyzed by mass spectrometry using the additive according to the present invention is not particularly limited, and may be, for example, a molecule having a molecular weight of 500 to 30,000, preferably 1,000 to 10,000. The additive according to the present invention is preferably used for mass spectrometry of a hydrophobic substance. In this case, a sample may contain a substance (e.g., a hydrophilic substance) other than the hydrophobic substance that is an analyte. The degree of hydrophobicity of the hydrophobic substance is not particularly limited as long as it is at a level that can be regarded as hydrophobic based on any well-known hydrophobicity index or hydrophobicity calculation method. For example, the degree of hydrophobicity of the hydrophobic substance may be at a level that can be regarded as hydrophobic by those skilled in the art based on the BB Index (Bull and Breese Index). More specifically, the BB Index of the hydrophobic substance may be, for example, 1000 or less, preferably -1000 or less.

Alternatively, the degree of hydrophobicity of the hydrophobic substance may be at a level that can be regarded as hydrophobic by those skilled in the art based on the HPLC Index. The HPLC Index is a hydrophobicity index reported by C.A. Browne, H.P.J. Bennett, and S. Solomon in Analytical Biochemistry, 124, 201-208, 1982, and is also referred to as "HPLC/HFBA retention" because it is based on retention time in reversed-phase HPLC using, as an eluent, an aqueous acetonitrile solution containing 0.13% heptafluoro-n-butyric acid (HFBA). More specifically, the HPLC Index of the hydrophobic substance may be, for example, 100 or more, for example, 100 to 10,000, preferably 100 to 1,000.

Particularly, the additive according to the present invention is highly effective in enhancing the ability of a matrix to ionize a hydrophobic peptide (in the present invention, the term "peptide" includes proteins). The determination as to whether a peptide to be analyzed is hydrophobic or not may be made based on the BB Index or the HPLC Index. More specifically, the hydrophobic peptide may be a peptide containing, as constituent amino acids, more amino acids having a higher degree of hydrophobicity. Examples of such hydrophobic amino acids include isoleucine, leucine, valine, alanine, phenylalanine, proline, methionine, tryptophan, and glycine. Further, cysteine, tyrosine, and the like may also be included.

The hydrophobic peptide may be a peptide having not only such a primary structure but also a higher-order structure with a higher degree of hydrophobicity. Examples of such a hydrophobic peptide include peptides having a structure likely to interact with the surface of a hydrophobic stationary phase in a reversed-phase HPLC column.

### [5. Formation of Crystal for Mass Spectrometry]

A crystal for mass spectrometry can be obtained through the step of forming, on a target plate for mass spectrometry, a liquid droplet of a mixture containing, in a solvent, at least an analyte, a matrix, and the additive according to the present invention and the step of removing the solvent from the formed liquid droplet of the mixture to obtain non-volatile matter contained in the mixture (i.e., at least the analyte, the matrix, and the additive) as a residue. The thus obtained residue is a crystal for mass spectrometry. In this specification, the term "crystal for mass spectrometry" is synonymous with the term "residue".

As the target for mass spectrometry, a conductive metal plate usually used in MALDI mass spectrometry may be used. More specifically, a plate made of stainless steel or gold may be used.

### [5-1. Preparation of Liquid Droplet of Mixture on Target Plate]

A specific method for preparing a liquid droplet of the mixture on a target plate is not particularly limited. For example, first, a sample solution containing an analyte, a matrix solution, and an additive solution are prepared separately from one another, or a sample solution containing an analyte and an additive-containing matrix solution are prepared separately from each other. Then, these solutions are mixed to obtain a mixture, and the obtained mixture is dropped onto a target plate. Alternatively, these solutions may be mixed on a target plate by dropping these solutions onto the same position on the target plate (on-target mix) . In the case of on-target mix, the order of dropping the solutions is not particularly limited.

The ratio of the additive to the matrix may be, for example, a molar ratio of 0.01 to 50 moles, preferably 0.05 to 0.5 moles per mole of the matrix.

More specifically, the additive may be prepared as, for example, a solution of 0.5 to 50 mg/mL, preferably 5 to 10 mg/mL, for example, 5 mg/mL. The matrix may be prepared as, for example, a solution of 1 mg/mL to a saturated concentration, preferably 1 to 10 mg/mL, for example, 10 mg/mL. For example, these additive solution and matrix solution may be mixed in a volume ratio of 10 : 1 to 1 : 10, preferably 1 : 1 to 1 : 10, for example 1 : 10.

The solvent of the mixture may be selected from the group consisting of acetonitrile (ACN), trifluoroacetic acid (TFA), methanol (MeOH), ethanol (EtOH), tetrahydrofuran (THF), dimethylsulfoxide (DMSO), and water. Specific examples of the solvent of the mixture include an aqueous ACN-TFA solution, an aqueous ACN solution, an aqueous MeOH-TFA solution, an aqueous MeOH solution, an aqueous EtOH-TFA solution, an aqueous EtOH solution, an aqueous THF-TFA solution, an aqueous THF solution, an aqueous DMSO-TFA solution, and an aqueous DMSO solution. Among them, an aqueous ACN-TFA solution or an aqueous ACN solution may be preferably used. The concentration of ACN in the aqueous ACN-TFA solution may be, for example, 10 to 90 vol%, preferably 25 to 75 vol%, and the concentration of TFA in the aqueous ACN-TFA solution may be, for example, 0.05 to 1 vol%, preferably 0.05 to 0.1 vol%.

The volume of the liquid droplet of the mixture is not particularly limited, and may be appropriately determined by those skilled in the art . When a well is provided on the target plate, the liquid droplet of the mixture may be formed in the well. In this case, the liquid droplet is formed so as to have a volume that can be held in the well. More specifically, the liquid droplet may be formed so as to have a volume of about 0.1 µL to 2 µL, for example, about 0.5 µL. [5-2. Removal of Solvent and Embodiment of Crystal for Mass Spectrometry]

The solvent is removed from the liquid droplet of the mixture on the target plate. The removal of the solvent includes natural evaporation of the solvent. The amount of the matrix contained per residue (that is, per crystal for mass spectrometry) generated by evaporation may be, for example, 1 to 1,000 nmol, preferably 10 to 100 nmol. As described above, the amount of the additive may be 0.01 to 50 times, preferably 0.05 to 0.5 times the amount of the matrix. The amount of the analyte may be in the range of 50 amol to 100 pmol or in the range of 100 amol to 50 pmol with respect to 25 nmol of the matrix.

The residue has a substantially circular shape on a surface in contact with the target plate. That is, the outer edge of the residue is substantially circular. The average diameter of the substantially circular shape may vary depending on the amount of the sample, the volume of the liquid droplet, the amount of the matrix, the composition of the solvent etc., but is for example 1 to 3 mm, preferably 1 to 2 mm. It is to be noted that the average diameter is the average of the lengths of line segments cut from lines passing through the center of gravity of the substantially circular shape by the outer edge of the residue. In the liquid droplet of the mixture prepared on the target plate, a hydrophobic substance is uniformly present before the solvent is removed. However, the hydrophobic substance is not uniformly present in the residue obtained by removing the solvent but is localized in the outer peripheral region of the residue. More specifically, the hydrophobic substance is enriched in the outer peripheral region of the residue, and is therefore less likely to be present in a region inside the outer peripheral region of the residue. More specifically, 50 mol% or more, preferably 70 mol% or more of the entire hydrophobic substance present in the residue may be localized in the outer peripheral region of the residue.
The outer peripheral region of the residue where the hydrophobic substance is localized has a substantially annular (ring) shape. The average diameter of the outer edge of the outer peripheral region, that is, the average outer diameter of the outer peripheral region is the average of the lengths of line segments cut from lines passing through the center of gravity by the outer edge of the outer peripheral region, which is in agreement with the average diameter of the residue that has already been described. On the other hand, the average diameter of the inner edge of the outer peripheral region, that is, the average inner diameter of the outer peripheral region is the average of the lengths of line segments cut from lines passing through the center of gravity by the inner edge of the outer peripheral region, which is, for example, 80% or more, preferably 90% or more of the average outer diameter. The upper limit of the above range is not particularly limited, but is, for example, 99%.

When a substance (e.g., a hydrophilic substance) other than the hydrophobic substance is contained in the sample, as described above, the hydrophobic substance is locally present in the residue, whereas the substance other than the hydrophobic substance is uniformly present in the entire region of the residue including the central part of the residue in which the hydrophobic substance is less likely to be present. In such a case, the substance other than the hydrophobic substance is relatively more hydrophilic than the hydrophobic substance. More specifically, the substance other than the hydrophobic substance may be a substance whose BB Index is larger than -1,000, preferably larger than 1, 000 or whose HPLC Index is smaller than 100, preferably smaller than 60.

Such localization of the hydrophobic substance is advantageous in that the hydrophobic substance as the analyte can be localized in a very small region. The localization of the analyte in a very small region makes it possible for the analyte to be densely present at a point irradiated with laser, which is preferred from the viewpoint of achieving sensitive analysis.

Therefore, sensitive mass spectrometry can be achieved not by irradiating the entire region of the residue (i.e., both the outer peripheral region and the region inside the outer peripheral region) with laser in a conventional manner but by irradiating only the ring-shaped outer peripheral region of the residue where the hydrophobic substance is localized with laser. However, the present invention does not particularly exclude the operation of irradiating the entire region of the residue with laser in a conventional manner in mass spectrometry. Even when the operation of irradiating the entire region of the residue with laser is performed, sensitivity-improving effect can be obtained by the effect of concentration of the hydrophobic substance in the outer peripheral region.

### [6. Mass Spectrometer]

A mass spectrometer using the additive according to the present invention is not particularly limited as long as it is used in combination with a MALDI (Matrix-Assisted Laser Desorption/Ionization) ion source. Examples of such a mass spectrometer include MALDI-TOF (Matrix-Assisted Laser Desorption/Ionization-Time-of-Flight) mass spectrometers, MALDI-IT (Matrix-Assisted Laser Desorption/Ionization-Ion Trap) mass spectrometers, MALDI-IT-TOF (Matrix-Assisted Laser Desorption/Ionization-Ion Trap-Time-of-Flight) mass spectrometers, and MALDI-FTICR (Matrix-Assisted Laser Desorption/Ionization-Fourier Transform Ion Cyclotron Resonance) mass spectrometers.

### Examples

Hereinbelow, the present invention will be described more specifically with reference to examples, but is not limited to the following examples.

### [Example 1: Synthesis of Additive Compound]

### [1-1. Ortho-Substituted Compound]

A mixture of 2,5-dihydroxybenzoic acid (10 mmol) 1, 1-bromooctane (12 mmol) 2, anhydrous K₂CO₃ (3.0 g), and anhydrous acetone (20 mL) was heated under reflux with stirring for 8 hours.

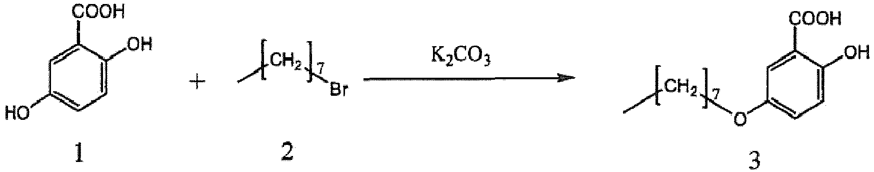

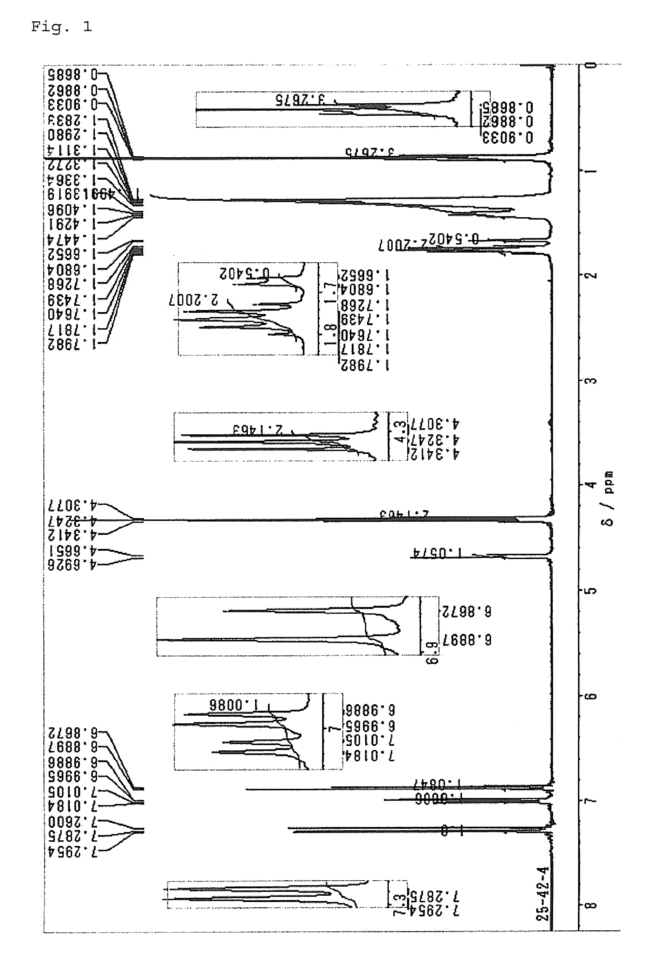

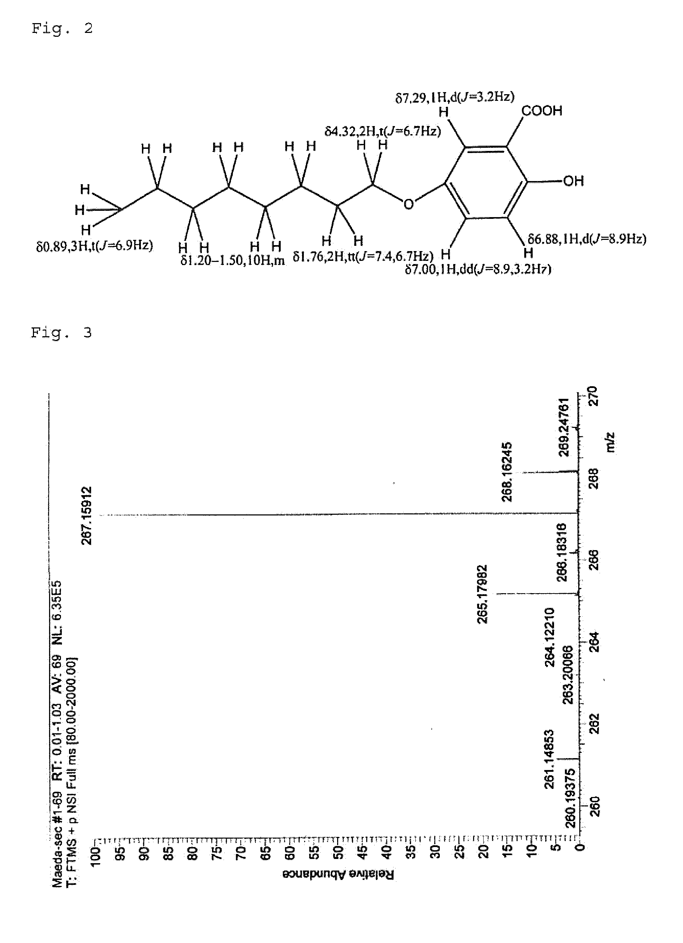

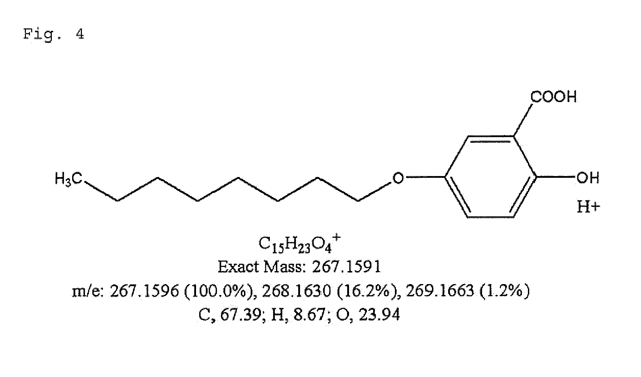

The mixture was cooled, and then the solvent was distilled away under a reduced pressure. Then, water (80 mL) was added thereto and extraction was performed with ether (100 mL) twice. The extracted ether layers were mixed and dried with Na₂SO₄. A target compound 3 was purified by silica gel column chromatography (ϕ 0.06-0.2 mm, SiO₂ 150 g, hexane : ethyl acetate = 8 : 2 (v/v), 70 cm column). The yield was 76% (which is about 70 to 85% on average and is lowered by about 10% by recrystallization). The compound was identified by ¹H NMR (400 MHz, CDCl₃) δ 7.29 (d, J = 3.2 Hz, 1H), 7.00 (dd, J = 8.9, 3.2 Hz, 1H), 6.88 (d, J = 8.9 Hz, 1H), 4.32 (t, J = 6.7 Hz, 2H), 1.76 (tt, J = 7.4, 6.7 Hz, 2H), 1.25 (m, 10H), 0.89 (t, J = 6.9 Hz, 3H) and HRMS (linear ion trap (LIT) -Orbitrap MS) : Calculated for C₁₅H₂₃O₄⁺[M+H]⁺ 267.1591, found 267.1591. Fig. 1 is a ¹H NMR spectrum and Fig. 2 shows the result of proton assignment. Fig. 3 is a mass spectrum and Fig. 4 shows the result of mass spectrometry.

### [1-2. Meta-Substituted Compound]

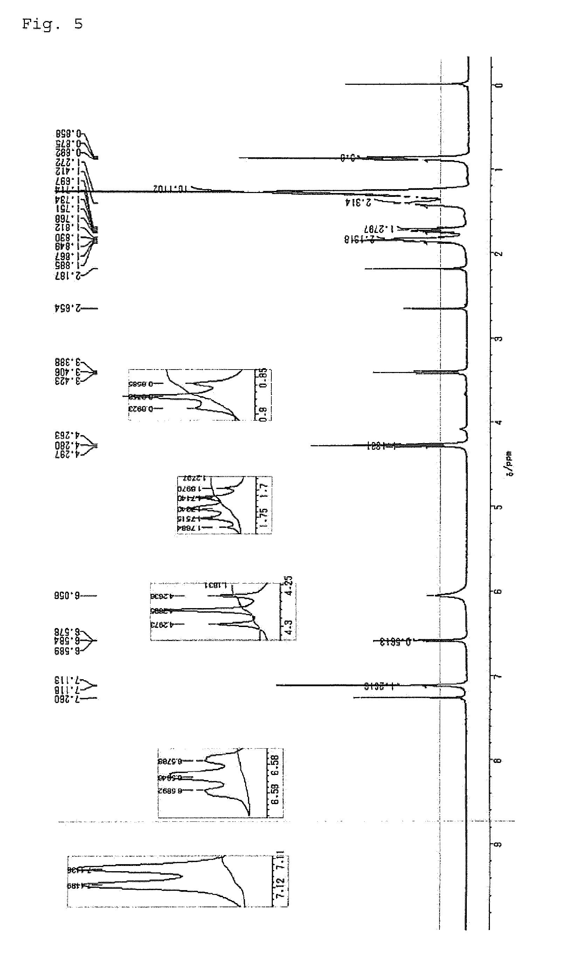

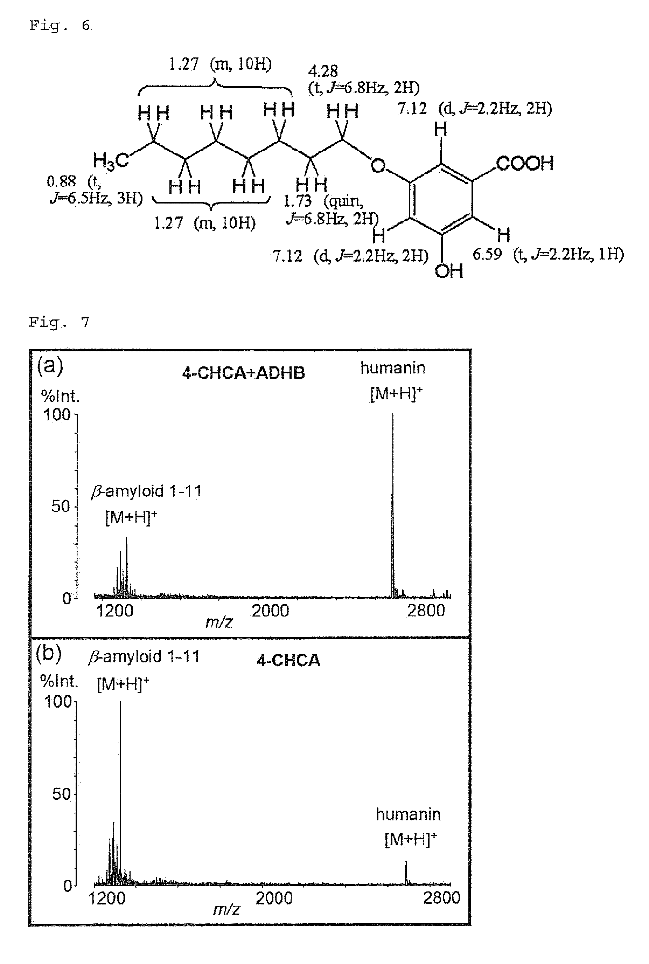

A mixture of 3,5-dihydroxybenzoic acid (10 mmmol), 1-bromooctane (12 mmol), NaOH (20 mmol), and anhydrous acetone (20 mL) was heated under reflux with stirring for 8 hours. The mixture was cooled, and then the solvent was distilled away under a reduced pressure. Then, water (80 mL) was added thereto and hydrochloric acid was added for pH adjustment (2 to 3), and then extraction was performed with ether (100 mL) twice. The extracted ether layers were mixed and dried with Na₂SO₄. A target compound was purified by silica gel column chromatography (ϕ 0.06-0.2 mm, SiO₂ 150 g, hexane : ethyl acetate = 8 : 2 (v/v), 70 cm column). The yield was 36%. The compound was identified by ¹H NMR (400 MHz, CDCl₃) δ 7.12 (d, J = 2.2 Hz, 2H), 6.59 (t, J = 2.2 Hz, 1H), 4.28 (t, J = 6.8 Hz, 2H), 1.73 (quin, J = 6.8 Hz, 2H), 1.27 (m, 10H), 0.88 (t, J = 6.5 Hz, 3H). Fig. 5 is a H NMR spectrum and Fig. 6 shows the result of proton assignment.

### [Example 2: Analysis of Hydrophobic Peptide (Humanin)]

In this example, a hydrophobic peptide (Humanin) was analyzed using the compound 3 (C8-ADHB) as an additive added to a matrix α-cyano-4-hydroxycinnamic acid (CHCA, hereinafter, also referred to as "4-CHCA"). For the purpose of comparison, the hydrophobic peptide was analyzed using a conventional matrix CHCA alone without using the additive.
(1) A 10 mg/mL 4-CHCA solution (50% ACN/0.05% TFA water (% is by volume; the same shall apply hereinafter)) and a 10 mg/mL C8-ADHB solution (50% ACN/0.05% TFA water) were mixed in a ratio of 10 : 1 (v/v) to prepare an additive-containing mixed matrix solution (C8-ADHB + CHCA).
(2) As sample solutions, 20 amol/µL to 2 pmol/µL solutions (50% ACN/0.05% TFA water) of a hydrophobic peptide Humanin were prepared.
(3) 0.5 µL of each of the sample solutions prepared in (2) and 0.5 µL of the matrix solution prepared in (1) were dropped into an individual well on a target plate (MALDI plate: sample plate with 2.8 mm ring X 384 wells and stainless-steel surface (Shimadzu/Kratos, UK); the same shall apply hereinafter) and mixed (on-target mix).
(4) Analysis was performed using AXIMA Performance (registered trademark) (Shimadzu Corporation) by linear TOF in positive and negative ion modes by manually searching a spot (sweet spot), allowing easy detection of sample ions, in a residue and irradiating the spot with laser. It is to be noted that in the following Examples 2 to 5, laser irradiation was performed in the same manner as in Example 1.

### (Comparative Example)

(1) As a matrix solution, a 10 mg/mL CHCA (Laser Bio) solution (50% ACN/0.05% TFA water) was prepared.
(2) As sample solutions, 20 amol/µL to 2 pmo2/µL solutions (50% ACN/0.05% TFA water) of a hydrophobic peptide Humanin were prepared.
(3) 0.5 µL of each of the sample solutions prepared in (2) and 0.5 µL of the matrix solution prepared in (1) were dropped into an individual well on a MALDI plate and mixed (on-target mix).
(4) Analysis was performed using AXIMA Performance (registered trademark) (Shimadzu Corporation) in linear mode, in positive and negative ion modes.

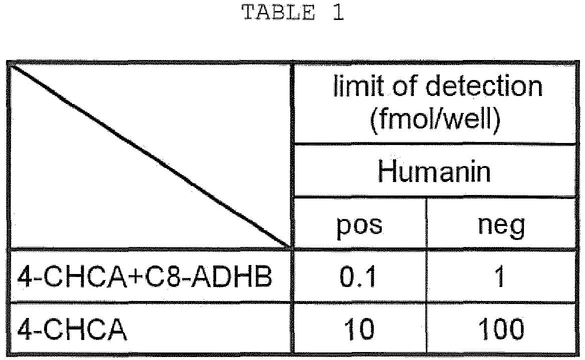

As can be seen from the above table, in both positive and negative ion modes, the limit of detection (mol/well) became two orders of magnitude lower, that is, sensitivity was improved 100 times when the additive-containing matrix (4-CHCA + C8-ADHB) was used as compared to when CHCA was used alone.

### [Example 3: Analysis of Various Hydrophobic Peptides (β-Amyloid 1-42, NF-κB inhibitor, [Gly14]-Humanin)]

### [3-1: Combination of Matrix CHCA and Additive C8-ADHB]

Various hydrophobic peptides were analyzed using the compound 3 (C8-ADHB) as an additive added to a matrix α-cyano-4-hydroxycinnamic acid (CHCA). For the comparison purpose, the hydrophobic peptides were analyzed using a conventional matrix α-cyano-4-hydroxycinnamic acid (CHCA) alone without using the additive.
(1) A 10 mg/mL 4-CHCA solution (50% ACN/0.05% TFA water) and a 10 mg/mL C8-ADHB solution (50% ACN/0.05% TFA water) were mixed in a ratio of 10 : 1 (v/v) to prepare an additive-containing matrix solution (C8-ADHB + GHCA).
(2) As sample solutions, 20 amol/µL to 2 pmol/µL solutions (50% ACN/0.05% TFA water) of each of the hydrophobic peptides, β-amyloid 1-42, NF-κB Inhibitor, and [Gly14]-Humanin were prepared.
(3) 0.5 µL of each of the sample solutions prepared in (2) and 0.5 µL of the matrix solution prepared in (1) were dropped into an individual well on a MALDI plate and mixed (on-target mix).
(4) Analysis was performed using AXIMA Performance (registered trademark) (Shimadzu Corporation) in linear mode, in positive and negative ion modes.

### (Comparative Example)

(1) As a matrix solution, a 10 mg/mL 4-CHCA (Laser Bio) solution (50% ACN/0.05% TFA water) was prepared.
(2) As sample solutions, 20 amol/µL to 2 pmol/µL solutions (50% ACN/0.05 TFA water) of each of the hydrophobic peptides, β-amyloid 1-42, NF-κB Inhibitor, and [Gly14]-Humanin were prepared.
(3) 0.5 µL of each of the sample solutions prepared in (2) and 0.5 µL of the matrix solution prepared in (1) were dropped into an individual well on a MALDI plate and mixed (on-target mix).
(4) Analysis was performed using AXIMA Performance (registered trademark) (Shimadzu Corporation) in linear mode, in positive and negative ion modes.

**TABLE 2**

| | limit of detection (fmol/well) | | | | | |
|---|---|---|---|---|---|---|
| | β-Amyloid 1-42 | | NF-kB Inhibitor | | [Gly14]-Humanin | |
| | pos | neg | pos | neg | pos | neg |
| 4-CHCA+C8-ADHB | 1 | 10 | 1 | 1 | 0.1 | 1 |
| 4-CHCA | 10 | 100 | 10 | 100 | 10 | 100 |

As can be seen from the above table, in both positive and negative modes, the limit of detection (mol/well) became one to two orders of magnitude lower, that is, sensitivity was improved 10 to 100 times when the additive-containing matrix (4-CHCA + C8-ADHB) was used as compared to when CHCA was used alone.

### [3-2: Combination of Matrix DHB and Additive C8-ADHB]

Various hydrophobic peptides were analyzed using the compound 3 (C8-ADHB) as an additive added to a matrix 2,5-dihydroxybenzoic acid (DHB). More specifically, analysis was performed in the same manner as in the above 3-1 except that the matrix was changed to DHB.

For the purpose of comparison, the hydrophobic peptides were analyzed using a conventional matrix 2, 5-dihydroxybenzoic acid (DHB) alone without using the additive. More specifically, analysis was performed in the same manner as in comparative example in the above 3-1 except that the matrix was changed to DHB.

**TABLE 3**

| | limit of detection (fmol/well) | | | | | |
|---|---|---|---|---|---|---|
| | β-amyloid 1-42 | | NF-kB Inhibitor, | | [Gly41]-Humanin | |
| | pos | neg | pos | neg | pos | neg |
| DHB+C8-ADHB | 1 | 10* | 1 | 10 | 1 | 10 |
| DHB | 1 | 10 | 10 | 100 | 10 | 100 |

As can be seen from the above table, in both positive and negative modes, sensitivity when the additive-containing matrix (DHB + C8-ADHB) was used was as high as that when the conventional matrix DHB was used alone. An asterisk besides the result indicates that S/N was increased. It was confirmed that, particularly in negative mode, the limit of detection (mol/well) became one order of magnitude lower, that is, sensitivity was improved 10 times.

### [Example 4: Analysis Using Various Additives (Influence of Chain Length of Hydrophobic Group on Sensitivity-Improving Effect)]

In this example, MALDI-TOF mass spectrometry was performed using a compound represented by the formula (I), where R has 4, 6, or 10 carbon atoms and the substituted carboxyl group and hydroxyl group are ortho to each other, in the same manner as in Example 2 to examine the influence of the chain length of the hydrophobic group R on sensitivity-improving effect.

The compound represented by the formula (I), where R has 4, 6, or 10 carbon atoms, was synthesized in the same manner as in Example 1 except that 1-bromooctane was changed to 1-bromobutane, 1-bromohexane, or 1-bromodecane, respectively.

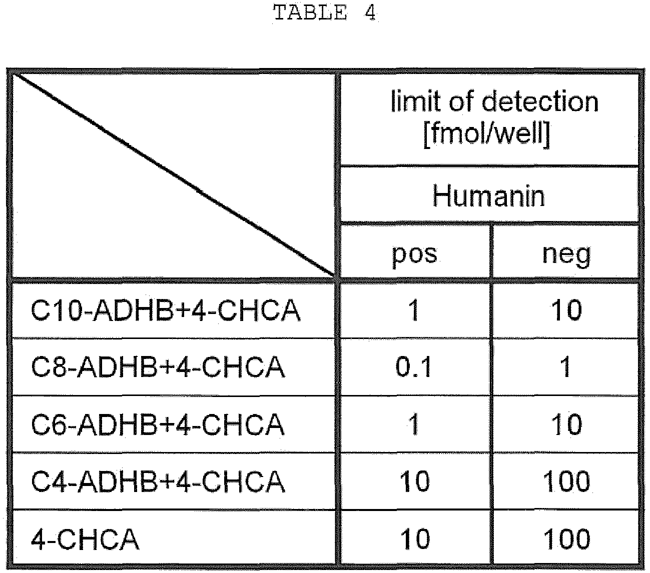

As can be seen from the above table, sensitivity was successfully improved also when the hydrophobic group R of the additive had 4, 6, 8, or 10 carbon atoms.

### [Example 5: Analysis Using Various Additives (Influence of Positional Isomerism on Sensitivity-Improving Effect)]

MALDI-TOF mass spectrometry was performed using, as the additive according to the present invention, a compound represented by the formula (I) where R has 8 carbon atoms and the substituted carboxyl group and hydroxyl group are ortho to each other, that is, the compound 3 (o-C8-ADHB) or a compound represented by the formula (I) where R has 8 carbon atoms and the substituted carboxyl group and hydroxyl group are meta to each other (m-C8-ADHB) in the same manner as in Example 2 to examine the influence of positional isomerism (ortho and meta) on sensitivity-improving effect.

**TABLE 5**

| | limit of detection [fmol/well] | |
|---|---|---|
| | Humanin | |
| | pos | neg |
| *m*-C8-ADHB+4-CHCA | 10 | 100 |
| *o*-C8-ADHB+4-CHCA | 0.1 | 1 |
| 4-CHCA | 10 | 100 |

Table 5 shows a comparison between the result of mass spectrometry using a matrix for mass spectrometry CHCA (α-cyano-4-hydroxycinnamic acid) and the additive in combination and the result of mass spectrometry without using the additive. As can be seen from the above table, sensitivity-improving effect, that is, detection limit-lowering effect was obtained when the ortho-substituted additive (o-C8-ADHB) was used.

The same experiments were performed using other matrices for mass spectrometry, DHB (2,5-dihydroxybenzoic acid), SA (sinapic acid), and DAN (1, 5-diaminonaphthalene). The results are shown in the following table. An asterisk besides the result indicates that an S/N ratio was improved.

As can be seen from the following table, the additive according to the present invention had sensitivity-improving effect (i.e., detection limit-lowering effect) and/or S/N ratio-improving effect.

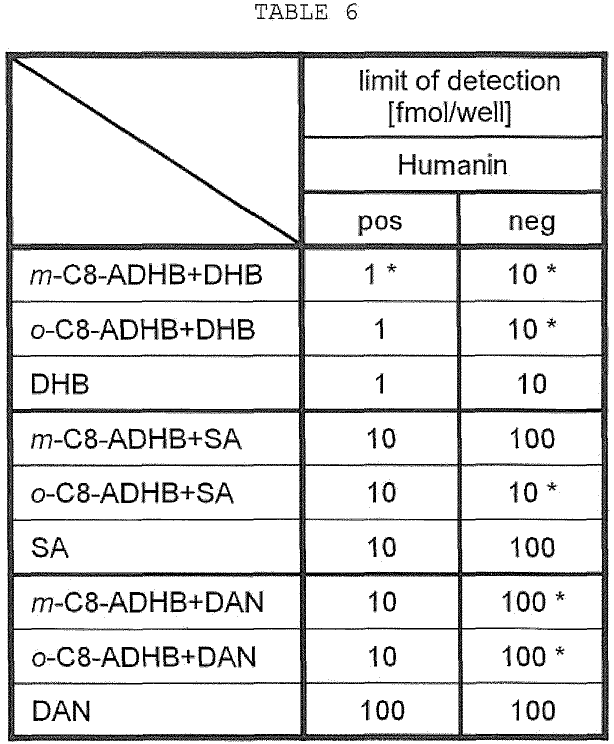

### [Example 6: Analysis of Mixture of Hydrophilic Peptide and Hydrophobic Peptide]

A 1 : 1 (mol/mol) mixture of a hydrophilic peptide β-amyloid 1-11 (BB Index: +2510, HPLC Index: 1.4) and a hydrophobic peptide Humanin (BB Index: -5800, HPLC Index: 117.4) was analyzed using the compound 3 (C8-ADHB) as an additive added to a matrix α-cyano-4-hydroxycinnamic acid (CHCA).

For the purpose of comparison, the mixture was analyzed using a conventional matrix α-cyano-4-hydroxycinnamic acid (CHCA) alone without using the additive.
(1) An additive-containing matrix solution (4-CHCA + ADHB) was prepared by mixing a 10 mg/mL 4-CHCA solution (50% ACN/0.05% TFA water) and a 5 mg/mL C8-ADHB solution (50% ACN/0.05% TFA water) in a ratio of 10 : 1 (v/v).
(2) As sample solutions, 1 : 1 (mol/mol) mixtures of a 20 amol/µL to 2 pmol/µL solution (50% ACN/0.05% TFA water) of a hydrophilic peptide β-amyloid 1-11 and a 20 amol/µL to 2 pmol/µL solution (50% ACN/0.05% TFA water) of a hydrophobic peptide Humanin were prepared.
(3) 0.5 µL of each of the sample solutions prepared in (2) and 0.5 µL of the matrix solution prepared in (1) were dropped into an individual well on a MALDI plate and mixed (on-target mix).
(4) Analysis was performed using AXIMA Performance (registered trademark) (Shimadzu Corporation) in linear mode, in positive and negative ion modes. More specifically, analysis was performed by raster scanning an area of 4000 µm × 4000 µm including the entire region of a residue in a well at 50 µm intervals (i.e., by automatically irradiating points in a certain region of a residue with laser at regular intervals) so that a total of 6561 points (81 × 81 points) were irradiated with two shots of laser.

### (Comparative Example)

(1) As a matrix solution, a 10 mg/mL 4-CHCA (Laser Bio) solution (50% ACN/0.05% TFA water) was prepared.
(2) As a sample solution, a 1 : 1 (mol/mol) mixture of a 20 fmol/µL solution (50% ACN/0.05% TFA water) of a hydrophilic peptide β-amyloid 1-11 and a 20 fmol/µL solution (50% ACN/0.05% TFA water) of a hydrophobic peptide Humanin was prepared.
(3) 0.5 µL of the sample solution prepared in (2) and 0.5 µL of the matrix solution prepared in (1) were dropped into an individual well on a MALDI plate and mixed (on-target mix).
(4) Analysis was performed using AXIMA Performance (registered trademark) (Shimadzu Corporation) in linear mode, in positive and negative ion modes.

### (Result 1)

Fig. 7 shows the results of analysis of the 1 : 1 mixture of a 20 fmol/µL solution of a hydrophilic peptide β-amyloid 1-11 and a 20 fmol/µL solution of a hydrophobic peptide Humanin (i.e., the results of analysis of a mixture of 10 fmol of the hydrophilic peptide and 10 fmol of the hydrophobic peptide placed on the MALDI plate). More specifically, Fig. 7 (a) is a positive mode mass spectrum obtained using the additive-containing matrix (4-CHCA + ADHB) and Fig. 7 (b) is a positive mode mass spectrum obtained using the matrix (4-CHCA) alone. When 4-CHCA was used alone, the hydrophilic peptide β-amyloid 1-11 was detected as a main peak, and the relative intensity of the peak of the hydrophobic peptide Humanin to that of the hydrophilic peptide was significantly low. On the other hand, when the additive-containing matrix was used, the hydrophobic peptide Humanin was detected as a main peak, and the relative intensity of the peak of the hydrophilic peptide β-amyloid 1-11 to that of the hydrophobic peptide was significantly low.

The results of analysis in negative mode showed the same tendency as described above.

### (Result 2)

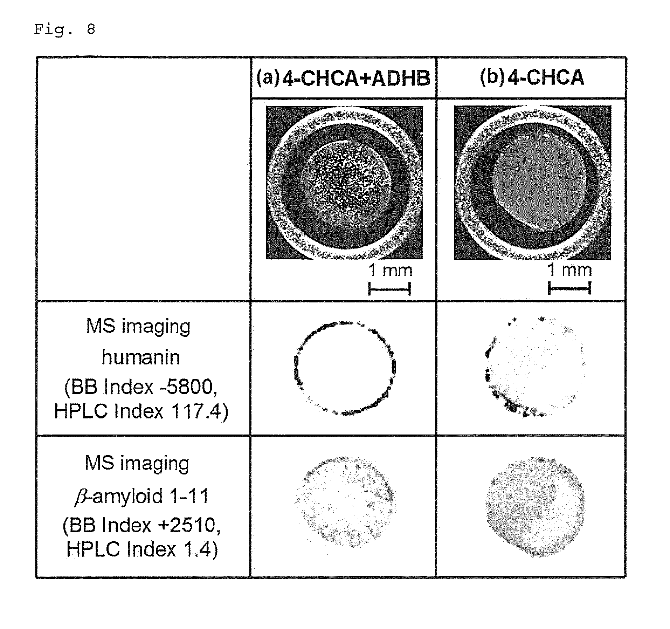

Fig. 8 shows a photograph of a crystal in a well on the MALDI plate, an MS image of the hydrophobic peptide Humanin analyzed in positive mode, and an MS image of the hydrophilic peptide β-amyloid 1-11 analyzed in positive mode, which were obtained when the additive-containing matrix 4-CHCA + ADHB was used (a) and those obtained when the matrix 4-CHCA was used alone (b).

When the additive-containing matrix 4-CHCA + ADHB was used, the hydrophilic peptide β-amyloid 1-11 was detected mainly in the inner region of the crystal and the hydrophobic peptide Humanin was detected mainly in the outer peripheral region of the crystal. On the other hand, when the matrix 4-CHCA was used alone, both the hydrophilic peptide and the hydrophobic peptide were detected in the entire region of the crystal.

Therefore, it can be considered that the presence of the additive has caused the localization of the hydrophobic peptide in the outer peripheral region in the process of sample-matrix crystallization. The enrichment effect of the hydrophobic peptide due to localization can be regarded as one of the possible causes of improved sensitivity for detection of the hydrophobic peptide in this example.

### [Example 7: Analysis of Protein Digests]

A tryptic digest of a protein, Phosphorylase b, was analyzed using the compound 3 (C8-ADHB) as an additive added to a matrix α-cyano-4-hydroxycinnamic acid (4-CHCA).

For the purpose of comparison, the tryptic digest was analyzed using a conventional matrix α-cyano-4-hydroxycinnamic acid (4-CHCA) alone without using the additive.
(1) An additive-containing matrix solution (4-CHCA + ADHB) was prepared by mixing a 10 mg/mL 4-CHCA solution (50% ACN/0. 05% TFA water) and a 5 mg/mL C8-ADHB solution (50% ACN/0.05% TFA water) in a ratio of 10 : 1 (v/v).
(2) As a sample solution, a 100 fmol/µL solution (50% ACN/0. 05% TFA water) of a commercially-available tryptic digest of Phosphorylase b (MassPREP™ phosphorylase b digestion standard, Waters) was prepared.
(3) 0.5 µL of the sample solution prepared in (2) and 0.5 µL of the matrix solution prepared in (1) were dropped into an individual well on a MALDI plate and mixed (on-target mix).
(4) Analysis was performed using AXIMA Performance (registered trademark) (Shimadzu Corporation) in linear mode, in positive ion mode. More specifically, analysis was performed by raster scanning an area of 4000 µm x 4000 µm including the entire region of a residue in a well at 50 µm intervals (i.e., by automatically irradiating points in a certain region of a residue with laser at regular intervals) so that a total of 6561 points (81 × 81 points) were irradiated with two shots of laser.

### (Comparative Example)

(1) As a matrix solution, a 10 mg/mL 4-CHCA (Laser Bio) solution (50% ACN/0.05% TFA water) was prepared.
(2) As a sample solution, a 100 fmol/µL solution (50% ACN/0.05% TFA water) of a tryptic digest of Phosphorylase b (MassPREP™ phosphorylase b digestion standard, Waters) was prepared.
(3) 0.5 µL of the sample solution prepared in (2) and 0.5 µL of the matrix solution prepared in (1) were dropped into an individual well on a MALDI plate and mixed (on-target mix).
(4) Analysis was performed using AXIMA Performance (registered trademark) (Shimadzu Corporation) by linear TOF in positive ion mode.

### (Result)

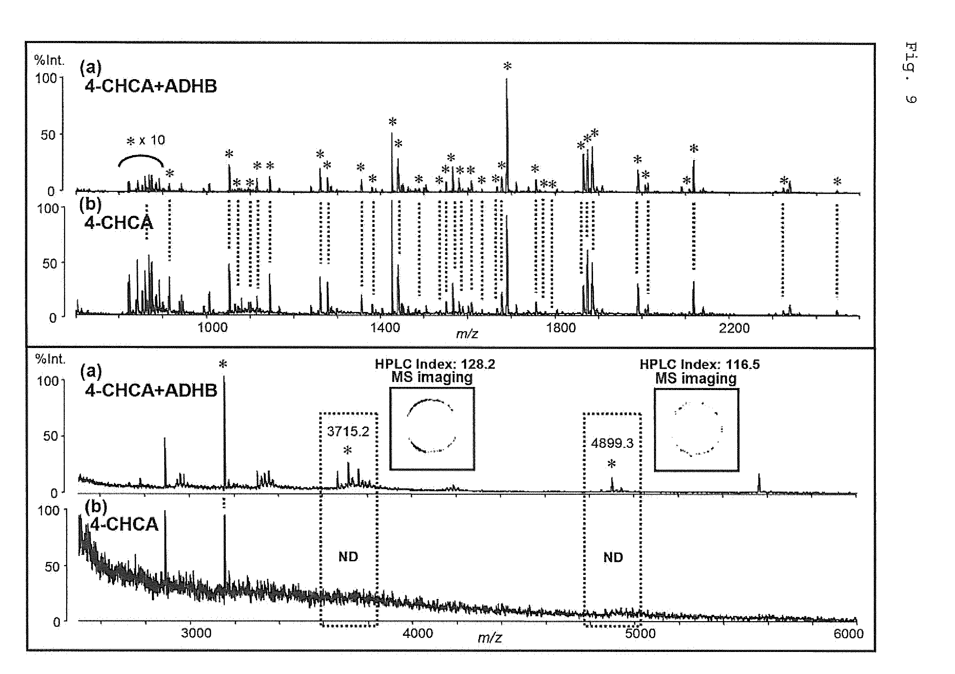

Fig. 9(a) is a mass spectrum of the tryptic digest of Phosphorylase b, which was obtained when the additive-containing matrix 4-CHCA + ADHB was used and Fig. 9 (b) is a mass spectrum of the tryptic digest of Phosphorylase b, which was obtained when the matrix 4-CHCA was used alone. An asterisk besides an ion peak indicates that the ion peak is derived from a peptide fragment of the tryptic digest of Phosphorylase b. ND indicates that no ions could be detected.

As shown in Fig. 9, the same ions were detected in the range of m/z 2500 or less in both cases where 4-CHCA + ADHB was used and where 4-CHCA was used, but in the range of m/z 2600 or more, peak intensities derived from peptide fragments were particularly high when ADHB was used. More specifically, ion peaks at m/z 3715.2 and m/z 4899.3 were detected only when C8-ADHB was used. In Fig. 9, MS images of peak substances at m/z 3715.2 and m/z 4899.3 are inserted.

From the MS images inserted in Fig. 9, it was confirmed that ions at m/z 3715.2 and m/z 4899.3 were detected in the outer peripheral region of a matrix-sample mixture crystal. That is, as in the case of Example 6 shown in Fig. 8, the enrichment effect of the peptide fragments due to localization in the outer peripheral region of a sample-matrix crystal can be regarded as one of the possible causes of improved sensitivity for detection of the peptide fragments.

As a result, the sequence coverage of the tryptic digest of Phosphorylase b was increased by about 10% when 4-CHCA + ADHB was used as compared to when 4-CHCA was used alone.

### [Example 8: Rate of Sensitivity Improvement by Using C8-ADHB]

Fourteen kinds of peptides different in HPLC Index (HPLC Index: -60.2 to 200.0) were prepared as analytes and analyzed using the compound 3 (C8-ADHB) as an additive added to a matrix α-cyano-4-hydroxycinnamic acid (CHCA) to determine how many times detection sensitivity would be improved as compared to when a conventional matrix α-cyano-4-hydroxycinnamic acid (4-CHCA) was used alone (i.e., to determine a sensitivity improvement rate).
(1) An additive-containing matrix solution (4-CHCA + ADHB) was prepared by mixing a 10 mg/mL 4-CHCA solution (50% ACN/0.05% TFA water) and a 5 mg/mL C8-ADHB solution (50% ACN/0.05% TFA water) in a ratio of 10 : 1 (v/v).
(2) As sample solutions for evaluating the limit of detection, 20 amol/µL to 2 pmol/µL solutions (50%ACN/0.05% TFA water) of each of the peptides, Temporin A amide NF-κB inhibitor, OVA-BIP hybrid peptide, melittin honey bee, β-amyloid 22-42, MPGΔNLS, β-amyloid 1-11, β-amyloid 1-28, GPHRSTPESRAAV (SEQ ID No. 1), β-conglycinin, Humanin, [Gly14]-humanin, β-amyloid 1-42, and ACTH 18-39 were prepared. On the other hand, as a sample for evaluating MS imaging, a sample mixed solution was prepared by mixing the above-mentioned 14 kinds of peptides in equimolar amounts.
(3) 0.5 µL of each of the sample solutions prepared in (2) and 0.5 µL of the matrix solution prepared in (1) were dropped into an individual well on a MALDI plate and mixed (on-target mix).
(4) Analysis was performed using AXIMA Performance (registered trademark) (Shimadzu Corporation) in linear mode, in positive ion mode. The limit of detection was measured by manually searching a spot (sweet spot), allowing easy detection of sample ions, in a residue and irradiating the spot with laser. MS imaging was performed by raster scanning an area of 4000 µm × 4000 µm including the entire region of a residue in a well at 50 µm intervals (i.e., by automatically irradiating points in a certain region of a residue with laser at regular intervals) so that a total of 6561 points (81 × 81 points) were irradiated with two shots of laser.

### (Comparative Example)

(1) As a matrix solution, a 10 mg/mL 4-CHCA (Laser Bio) solution (50% ACN/0.05% TFA water) was prepared.
(2) As sample solutions, 20 amol/µL to 2 pmol/µL solutions (50%ACN/0.05% TFA water) of each of the peptides, Temporin A amide, NF-κB inhibitor, OVA-BIP hybrid peptide, melittin honey bee, β-amyloid 22-42, MPGΔNLS, β-amyloid 1-11, β-amyloid 1-28, GPHRSTPESRAAV (SEQ ID No. 1), β-conglycinin, Humanin, [Gly14]-humanin, β-amyloid 1-42, and ACTH 18-39 were prepared.
(3) 0.5 µL of each of the sample solutions prepared in (2) and 0.5 µL of the matrix solution prepared in (1) were dropped into an individual well on a MALDI plate and mixed (on-target mix).
(4) Analysis was performed using AXIMA Performance (registered trademark) (Shimadzu Corporation) in linear mode, in positive ion mode.

### (Result)

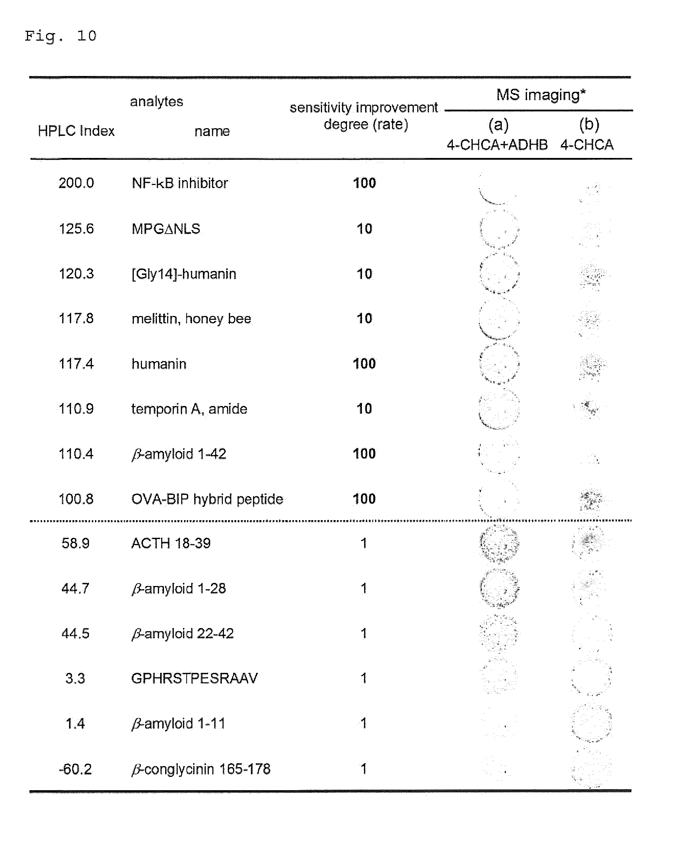

Fig. 10 shows the HPLC Index and name of each of the analytes, the sensitivity improvement degree (rate) when 4-CHCA + ADHB was used (a) as compared to when the conventional matrix α-cyano-4-hydroxycinnamic acid (4-CHCA) was used alone (b), and positive mode MS images of each of the analytes obtained when 4-CHCA + ADHB was used (a) and when 4-CHCA was used alone (b). The MS images were created based on the relative intensities of [M+H]⁺ ion peaks of the analytes.

As shown in Fig. 10, it was confirmed that the additive C8-ADHB had sensitivity-improving effect mainly on the hydrophobic peptides having an HPLC Index of 100 or more among the 14 kinds of peptides. Further, it was confirmed from the MS images obtained when 4-CHCA + ADHB was used that all the peptides having an HPLC Index of 100 or more were detected in the outer peripheral region of a sample-matrix crystal. As in the cases of Example 6 shown in Fig. 8 and Example 7 shown in Fig. 9, the enrichment effect of the peptide fragments due to localization in the outer peripheral region of a sample-matrix crystal can be regarded as one of the possible causes of improved sensitivity for detection of the peptide fragments.

## Claims

1. A matrix additive for mass spectrometry, which is 5-alkoxy-2- or -3-hydroxybenzoic acid represented by the following formula (I): 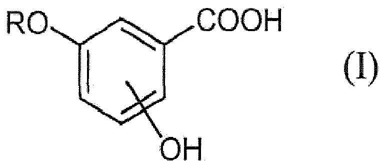 where R is an alkyl group having 6 to 10 carbon atoms and the substituted carboxyl group and hydroxyl group are ortho or meta to each other.

2. The additive according to claim 1, which is added to a matrix for mass spectrometry selected from the group consisting of α-cyano-4-hydroxycinnamic acid, 2, 5-dihydroxybenzoic acid, sinapic acid, and 1,5-diaminonaphthalene.

3. The additive according to claim 1, which is used for mass spectrometry of a hydrophobic substance.

4. The additive according to claim 3, wherein the hydrophobic substance is a hydrophobic peptide.

5. The additive according to claim 3, wherein the hydrophobic substance has an HPLC Index of 100 to 10,000.

6. The additive according to claim 1, which is used in a molar ratio of 0.01 to 50 moles per mole of a matrix for mass spectrometry.

7. A method for forming a crystal for mass spectrometry, comprising the steps of: preparing, on a target plate for mass spectrometry, a liquid droplet of a mixture containing, in a solvent, at least a hydrophobic substance, a matrix, and the additive according to clam 1; and removing the solvent from the liquid droplet.

8. A crystal for mass spectrometry formed on a target plate for mass spectrometry, the crystal for mass spectrometry comprising at least a hydrophobic substance, a matrix, and the additive according to claim 1 and having a substantially circular shape with an average diameter of 1 to 3 mm on a surface in contact with the target plate for mass spectrometry, wherein 50 mol% or more of the hydrophobic substance is localized in an outer peripheral region of the substantially circular shape, the average diameter is an average outer diameter of the outer peripheral region, and the outer peripheral region has an average inner diameter that is 80% or more of the average outer diameter.

9. A mass spectrometry method comprising the steps of:
preparing, on a target plate for mass spectrometry, a liquid droplet of a mixture containing, in a solvent, at least a hydrophobic substance, a matrix, and the additive according to claim 1; removing the solvent from the liquid droplet to form a crystal for mass spectrometry; and irradiating the crystal for mass spectrometry with laser to detect the hydrophobic substance.

10. The mass spectrometry method according to claim 9, wherein an outer peripheral region of the crystal for mass spectrometry, whose average inner diameter is 80% or more of its average outer diameter, is irradiated with the laser.

11. The mass spectrometry method according to claim 9, wherein the mixture further contains a hydrophilic substance.

12. 5-Alkoxy-2- or -3-hydroxybenzoic acid represented by the following formula (I): 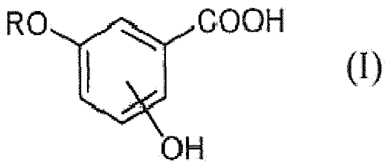 where R is an alkyl group having 6 to 10 carbon atoms and the substituted carboxyl group and hydroxyl group are ortho or meta to each other.
